# EUROPEAN PATENT APPLICATION

(11) **EP 1 229 331 A1**
(43) Date of publication of application: **07.08.2002**
(21) Application number: 01102300.9
(22) Date of filing: 01.02.2001
(51) Int. Cl.: G01N 33/68

(54) **Mass spectrometic detection of abnormal prion protein in the diagnosis of transmissible spongiform encephalopathies**

(71) Applicant: Krebs, Stefan, 85764 Oberschleissheim (DE); Förster, Martin, 81827 München (DE)
(72) Inventor: Krebs, Stefan, 85764 Oberschleissheim (DE); Förster, Martin, 81827 München (DE)

(57) **Abstract**

The invention comprises diagnosis of transmissible spongiform encephalopathies by taking a tissue sample, preferably blood from a living animal or human, or a food sample and detecting the presence or absence of abnormal prion protein by mass spectrometry. The invention is characterised by the introduction of a chemical modification of the prion protein that allows the distinction of the normal and abnormal, disease-related forms of said protein by mass spectrometry. In preferred embodiments these modifications are further characterised as H/D exchange or covalent modifications of exposed side-chains or backbone termini before or after protease digestion.

## Description

Transmissible spongiform encephalopathies (TSE's) are fatal neurodegenerative diseases that affect mammals including cattle (BSE), human (Creutzfeld-Jakob-Disease) and sheep (scrapie). The disease is characterised by the accumulation of an abnormal, protease-resistant form (PrP-res or PrP^{sc}) of a host-coded membrane-associated glycoprotein known as prion protein (PrP), in brain tissue. PrP-res is thought to be the infectious agent by itself without involving any nucleic acid component (Prusiner, *Science* 216:136-144) and can be transmitted by ingestion or surgery. It propagates the disease by transmitting its protease-resistant conformation to its properly folded functional counterpart ( termed PrP^{c} or PrP-sen), expressed on the surface of a variety of cell types, especially neuronal cells and leukocytes. This process is slow and PrP-res is present in the affected organism during a long pre-clinical incubation period until finally leading to the clinical phase of plaque-forming accumulation in the brain.

The three-dimensional solution structure of the normal, protease sensitive form (PrP-sen) of prion proteins from various species is known and it exists sufficient evidence that PrP-res represents an alternative conformation with a significantly enhanced beta-sheet content. The beta-sheet rich conformation is prone to aggregation and can form amyloid fibers accumulating in so-called amyloid plaques in brain tissue and leading to pathological alteration of the brain. The presented invention takes advantage of these differences in conformation by submitting a prion sample isolated from a mammal to a chemical reaction that leads to different products in dependence of prion protein conformation. The reaction products are then analysed in order to determine absence or presence of products specific for PrP-res.

The BSE tests currently in use detect the protease resistant form of the prion protein by western blotting or ELISA detection of a proteinase K digest of brain extracts. Prior art focuses on immunologic techniques, either by use of synthetic peptide for generation of antibodies (WO 99/15651) or by immunodetection of PrP-res (WO 00/29850; WO 00/48003). Another alternative has been reported by the measuring the expression level of a set of marker proteins that indicate an infection with a TSE (WO 00/65357). Regarding detection of prions with other than immunologic methods, only one application comprising optical detection on a dielectric waveguide-chip has been issued (EP 0887645). Mass spectrometrical investigations of the prion protein include characterisation of glycosylation (Rudd et al., *PNAS* 96:13044-9) and analysis and mapping of proteolytic digests (Stahl et al. *Biochemistry* 32:1991-2002; Baskakov et al. *Biochemistry* 39:2792-2804; Parchi et al.).

There is a recognised need for screening TSE's in the preclinical state by detection of infectous PrP^{sc} in live animals or human, which most practicably means from a blood sample. A method suitable for PrP^{sc} detection in blood must be of highest sensitivity, as the amount to detect might be as small as 1 pg, a requirement that immuno-detection might not be able to fulfil (Brown et al. *J Lab Clin Med* 137:5-13). Mass spectrometry, in contrast can deal with such low amounts, especially when used with the modifications presented by the invention.

According to the invention the method for detection of abnormal prion protein in the diagnosis of transmissible encephalopathies comprises
- a): collecting a tissue sample from an animal or human or a food sample
- b): isolating the prion protein from said sample by means of a solid-phase bound antibody
- c): performing a chemical modification of the isolated prion protein
- d): analysing the reaction products of said modification by mass spectrometry
- e): assessment of prion conformation by comparison of the analysed reaction products with controls
A TSE is diagnosed if products specific for the abnormal disease-related conformation are detected.

The tissue sample is most preferably blood. Preferably the blood sample is collected in vessels containing a anticoagulating agent, of which EDTA or heparin are preferred. Other collection techniques like biopsy are also possible. Preferred are tissues known to express high amounts of prion protein like tonsils, spleen and squamous epithelia of skin and gastrointestinal tract. Also preferred is a post-mortem analysis using central nervous or lymphoreticular tissue. Also preferred is unprocessed food, such as milk, meat or inner organs, as well as processed food.

The tissue sample containing prion protein has to be treated in order to enrich said protein. Preferred is centrifugation of a blood sample to obtain the buffy coat, a technique well known to the skilled practitioner. The isolated buffy coat has to be treated with a detergent in order to solubilise the membrane bound prion protein. For solubilisation of membrane proteins a vast variety of detergents are commonly in use (Bhairi, S. Detergents, Calbiochem Corp. 1997). Preferred are NP-40, Tween-20, Tween-80, n-octyl-β-D-glucopyranoside, n-heptyl-β-D-glucopyranoside, n-hexyl-β-D-glucopyranoside, n-nonyl-β-D-glucopyranoside, n-decyl-β-D-glucopyranoside, n-octyl-β-D-maltopyranoside, n-heptyl-β-D-maltopyranoside, n-hexyl-β-D-maltopyranoside, n-nonyl-β-D-maltopyranoside, n-decyl-β-D-maltopyranoside, n-dodecyl-β-D-maltopyranoside, n-octyl-p-D-thioglucopyranoside, deoxycholic acid, Triton x-100, Triton X-114, CHAPS, ZWITTERGENT, sodium laurylsarcosine, N,N-dimethyldodecylamine-N-oxide, C₈E₄, sulfobetaine SB 3-14. Treatment of the buffy coat with a detergent, which is used at a concentration above its critical micellar concentration, results in lysis of the cells and solubilisation of membrane bound proteins. The lysate is then added to an immobilised antibody. Immobilisation is achieved by covalent binding of an antibody to a solid phase. This can be a chromatography support or most preferred magnetic beads. The antibody must be capable of binding prion protein. Preferred is a monoclonal antibody, most preferred is 6H4. Isolation of prion protein is also possible by mixing buffy coat and the immobilised antibody first and then releasing the membrane bound proteins by solubilisation as described above or by treatment with a deglycosylating agent in order to cleave the GPI-anchor that attaches PrP to the membrane. Preferred deglycosylating agents are phosphatidylinositol phospholipase C or PNGase F. Alternatively PrP^{sc} can be extracted as described in pat. US6150172 or in pat. US6166187.

The isolated prion protein is then subjected to one or more chemical modifications. The purpose of said modifications is to distinguish between the two prion conformations Prp^{c} and PrP^{sc} as they yield different products. Another purpose is to enhance the sensitivity in mass spectrometrical detection by introduction of a permanently charged group or a group that is easily ionised during the process of ion generation (ESI or MALDI). By ease of ionisation we understand that ions are more effectively formed at the site of modification than in the unmodified protein. Modification that are preferred are derivatisation of the amino terminus or of amino acid side-chains with a charged or easily ionised moiety or hydrogen/deuterium exchange. In preferred embodiments these modifications are further characterised. The modifications can be made in solution after the prion protein has been released from the immobilized antibody or when it is still bound to it.

The chemically modified prion protein is then analysed by mass spectrometry using any known technique. Preferred is MALDI mass spectrometry in a high-throughput setting. As sensitivity is generally higher for smaller fragments the prion protein can be cleaved after the modification reaction. This can be achieved chemically, preferably by CNBr, or by proteolytic enzymes after denaturation of both PrP^{c} and PrP^{sc}. Preferably prion proteins are denatured by 8M Urea, 6M guanidinium hydrochloride or guanidinium thiocyanate.

In a preferred embodiment the prion proteins are isolated with a solid-phase bound antibody and subjected to H/D exchange by incubation with D₂O or an other appropriate deuterated solvent. Prior to hydrogen exchange the prion proteins can be derivatized with a permanently charged group to enhance the sensitivity in mass spectrometric analysis. Hydrogen exchange is performed under conditions where different extents of deuteration can be achieved for PrP-sen and PrP-res. Said conditions comprise the native solution conformations of both proteins, immunocomplexes or complexes to proteins capable of binding prion protein and denatured or partially unfolded states under conditions where PrP-sen is denatured or partially unfolded but PrP-res retains its native conformation. The latter is achieved by adding a denaturing agent. Preferred is 2M of guanidinium hydrochloride, but as unfolding is an equilibrium process it is conceivable that any concentration lying in the range of 0-4M will lead to similar results, differing in the total amount of exchanged protons in PrP^{c} but leaving sufficient difference between PrPc and PrPsc to distinguish both forms by their deuteration pattern. Also preferred are the denaturants guanidinium thiocyanate and urea. Regarding their concentration the above statement is valid as well and the whole range of 0-4 M is applicable.
The H/D exchange rate is then decreased or 'frozen' by change of the medium, e.g. by lowering the pH and/or temperature and/or drying of the sample and the sample is analysed in order to determine the extent of deuteration by mass spectrometry.

In another preferred embodiment the prion proteins are isolated with a solid-phase bound antibody and subjected to a chemical reaction that modifies the peptide chain termini and/or exposed reactive side-chains, with or without previous treatment by a proteolytic enzyme. The modification can be the introduction of one or more quarternary ammonium groups resulting in permanently positively charged molecules, thus altering the mass spectrometrical properties and especially in matrix-assisted laser desorption/ionisation mass spectrometry significantly enhancing the sensitivity. The modification can also be the introduction of a permanently charged aryl- or alkyl-substituted phosphonium residue. Preferably the amino groups of exposed lysine side-chains are reacted with an activated compound that introduces a fixed charge that facilitates detection of the reaction products. An activated compound can be a N-hydroxysuccinimide ester, an acid halogenide or an acid anhydride. The reaction is made under conditions where PrP-sen and PrP-res show different accessibility of reactive amino acid side chains due to differences in conformation. Without wishing to be bound by any theory we believe that such differences in side chain accessibility can just be due to the different native conformations of PrP-res and PrP-sen or they can be induced by the medium. Conditions inducing differential side-chain accessibility can be binding to a protein or presence of a denaturing agent in concentrations that completely or partially unfold PrP-sen, but keep PrP-res structurally intact.
The products of the above mentioned derivatisation are analysed by mass spectrometry either of the whole protein or fragments thereof, generated by proteolytic enzymes or chemical cleavage. A TSE is diagnosed if one or more products that are specific for the PrP-res conformation are detected.

A person skilled in the art can easily create variations of this method by adding or omitting single steps or changing their order or exchanging reagents that are commonly used in laboratory practice without deviating from the scope of the invention that consists in the creation and detection of a conformation-specific reaction product in order to assess prion protein conformation for a diagnostic purpose.

### Example 1: Detection of BSE by an altered deuteration pattern of the pathological beta-sheet conformation of prion protein

A blood sample taken from a live animal is centrifuged to separate the blood cells, the buffy coat is transferred to a new well, centrifuged and washed and the prion protein is solubilised with PBS containing 1% NP-40. After centrifugation to remove cell debris the supernatant is transferred to a fresh tube and 5 µl suspension of magnetic beads coupled with a monoclonal antibody are added. Solubilised prion protein is allowed to bind to the solid phase bound antibody by incubation for 1h at 4°C, the supernatant is removed and the beads are washed twice.

In order to lower the detection threshold the amino terminus of the prion protein is covalently modified with Tris[(2,4,6-trimethoxyphenyl)-phosphonium]acetic acid N-hydroxysuccinimide ester. For this purpose the beads with the bound prion protein are incubated for 20 min at 30°C with 10 µl of 1 mM Tris[(2,4,6-trimethoxyphenyl)-phosphonium]acetic acid N-hydroxysuccinimide ester in 50 mM phosphate buffer at pH 8.2. After completion of the reaction the beads are washed with 10 mM phosphate buffer pH 7.4.

To the drained magnetic particles 10 µl D₂O containing a denaturant are added and incubated for 10 min.

By addition of 2 µl 1% TFA in D₂O the prion protein is released from the antibody-beads and the hydrogen exchange rate is reduced by the low pH, thus conserving the deuteration state during the subsequent steps. The supernatant is immediately processed for MALDI analysis. Therefore 2 µl of supernatant are mixed with 2 µl of matrix solution prepared with D₂O/acetonitril. 1 µl of the mixture is spotted on the MALDI target and dried in a closed vessel containing dried silica in order to avoid back-exchange of deuterons with atmospheric water.

The sample is analysed in a MALDI mass spectrometer. In a sample of a BSE free animal only one peak corresponding to the maximally deuterated prion protein can be observed, whereas in BSE affected animals a second peak at lower mass corresponding to the partially exchange-refractory beta-sheet structure is observed. The presence of this lower mass peak is thus used for diagnosis of BSE.

### Example 2: Detection of BSE by covalent modification of exposed lysine side-chains

A blood sample taken from a live animal is centrifuged to separate the blood cells, the buffy coat is transferred to a new well, centrifuged and washed and the prion protein is solubilised with PBS containing 1% NP-40. After centrifugation to remove cell debris the supernatant is transferred to a fresh tube and 5 µl suspension of magnetic beads coupled with a monoclonal antibody are added. After a 1h incubation at 4°C supernatant is removed and the beads are washed twice.

To the drained magnetic particles 10 µl of PBS pH 9.5 containing 5mg/ml 6-trimethylammonium-hexyryl-N-hydroxy-succinimidyl-ester are added and incubated for 30 min at room temperature. After washing with PBS the derivatised prion proteins are released by addition of 1 µl 2% TFA and the supernatant is used directly for MALDI analysis. Alternatively the proteins can be denatured and cleaved into smaller peptidic fragments either by a protelytic enzyme or by CNBr cleavage.

1 µl of said supernatant is spotted on a dried droplet of α-cyano-4 hydroxy cinnamic acid methyl ester (0.5 µl of a 1% solution in acetone). In this non-ionizing matrix the reaction products carrying at least one permanent charge are detected with enhanced sensitivity. As the number of solvent accessible and therefore reactive lysine side-chains is dependent on the protein conformation, the m/z signals obtained in MALDI analysis can be addressed either to the normal or pathogenic form of the prion protein. Other matrices commonly used in MALDI mass spectrometry, such as sinapinic acid, α-cyano-4 hydroxy cinnamic acid or gentisic acid, can also be applied.

In MALDI mass spectrometry ions are normally formed in the gas phase by the matrix after the laser pulse. Normally the ion yield is low, depending on the chemistry of the analyte, and leads to a variety of singly and multiply charged anions and cations. The use of pre-formed ions by covalent modification of lysine side-chains, as presented herein, significantly lowers the detection limit, as literally all analyte molecules that are transferred to the gas phase by the laser pulse can be detected. This makes the method ideally suited for the detection of very small amounts of PrP^{sc} as in the early pre-clinical phase of BSE infections.

### Example 3: Detection of BSE by charge-derivatisation of the amino terminus of PrP-res

A blood sample taken from a live animal is centrifuged to separate the blood cells, the buffy coat is transferred to a new well, centrifuged and washed and the prion protein is solubilized with PBS containing 1% NP-40. After centrifugation to remove cell debris the supernatant is transferred to a fresh tube and 5 µl of magnetic beads coupled with a monoclonal antibody are added. After a 1h incubation at 4°C the supernatant is removed and the beads are washed twice with PBS.

The prion protein is partially digested by adding 10 µl of 50 mM phosphate at pH 8.2 and 2 u/ml proteinase K to the drained magnetic beads and incubated for 30 min at 47°C. The supernatant is transferred to a fresh tube and free amino termini of the resulting peptides are derivatized by addition of 2 µl of a 10 mM solution of Tris[(2,4,6-trimethoxyphenyl)-phosphonium]acetic acid N-hydroxysuccinimide ester. The mixture is incubated at 30°C for 20 min.

The protease resistant core is thus charge-labeled at the N-terminus, significantly lowering the detection limit in MALDI mass spectrometry by not depending on gas-phase ion formation during the desorption process, and can be detected directly as the only high molecular weight species of prion protein. The presence of PrP^{c} is assessed by the presence of specific, charge-labeled small degradation products and serves as a positive control for the whole process, as PrP^{c} is present in healthy as well as in affected animals.

## Claims

1. A method of diagnosis of pre-clinical or clinical transmissible spongiform encephalopathies comprising
a) collecting a tissue sample from an animal or human or a food sample
b) isolating the prion protein from said sample by means of a solid-phase bound antibody
c) performing a chemical modification of the isolated prion protein
d) analysing the reaction products of said modification by mass spectrometry
e) assessment of prion conformation by comparison of the analysed reaction products with controls

2. Method according to 1. where the chemical modification is H/D exchange.

3. Method according to 1. where the chemical modification is derivatisation of amino acid side-chains with a permanently charged moiety

4. Method according to 3. where the permanently charged moiety is a quarternary aliphatic ammonium group, NR₄

5. Method according to 3. where the permanently charged moiety is Tris[(2,4,6-trimethoxyphenyl)-phosphonium]acetyl

6. Method according to 1. where the chemical modification is derivatisation of amino acid side-chains with an easily ionised moiety that enhances the sensitivity in mass spectrometric detection

7. Method according to 6. where the easily ionised moiety is a substituted sulfonic acid, RSO₃H.

8. Method according to 1. where the chemical modification is enzymatic cleavage and derivatisation of the amino termini with a permanently charged moiety.

9. Method according to claims 2. to 8., where the chemical modification is followed by denaturation and enzymatic cleavage.

10. Method according to claims 2. to 8., where the chemical modification is followed by denaturation and chemical cleavage.

11. Method according to claims 2. to 10. where the sample is treated by a deglycosylating agent.
